(19) Europäisches Patentamt
European Patent Office
Office européen des brevets

(11) Numéro de publication : **0 338 898 B1**

(12) # FASCICULE DE BREVET EUROPEEN

(45) Date de publication du fascicule du brevet : **08.07.92 Bulletin 92/28**

(51) Int. Cl.$^5$ : **C07C 37/62,** C07C 39/27, C07C 41/22

(21) Numéro de dépôt : **89401027.1**

(22) Date de dépôt : **14.04.89**

(54) **Procédé de préparation d'un dérivé mono-bromé en ortho du phénol.**

(30) Priorité : **22.04.88 FR 8805329**

(43) Date de publication de la demande : **25.10.89 Bulletin 89/43**

(45) Mention de la délivrance du brevet : **08.07.92 Bulletin 92/28**

(84) Etats contractants désignés : **AT BE CH DE ES FR GB GR IT LI LU NL SE**

(56) Documents cités :
**GB-A- 2 175 895**
**US-A- 4 182 912**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière Route de Ternay**
**F-69360 Communay (FR)**
Inventeur : **Jouve, Isabelle**
**30, rue Léon Fabre**
**F-69100 Villeurbanne (FR)**
Inventeur : **Nonn, Alain**
**11, allée de la Gravière**
**F-69110 Sainte Foy les Lyon (FR)**

(74) Mandataire : **Dubruc, Philippe et al**
**RHONE-POULENC CHIMIE Service Brevets Chimie 25, quai Paul-Doumer**
**F-92408 Courbevoie Cédex (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne la préparation d'un dérivé mono-bromé en ortho du phénol de formule :

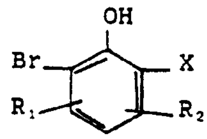

dans laquelle X représente OH,un radical alcoxy, $CH_2OH$, CHO, $CO_2R$, R étant un radical alkyle, alcényle, alkyloxy, ces radicaux pouvant être linéaire ou ramifiés, cycloalkyle, cycloalcényle, alkylcycloalcényle, benzyle, amine, terpénique, glycolique, $R_1$ et $R_2$ sont identiques ou différents et représentent l'hydrogène, un halogène, un radical alkyle, acyle ou alcoxy et avec comme condition que la position en ortho du OH soit libre, cette préparation se faisant à partir du produit de formule (1)

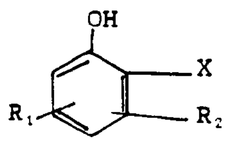
(1)

X, $R_1$ et $R_2$ etant tels que précédemment définis.

Le problème qui se pose pour la bromation de tels produits est double. On veut d'une part obtenir un dérivé monobromé et non pas dibromé. D'autre part, cette bromation doit être regio-sélective car on désire former essentiellement le dérivé bromé en ortho. On peut indiquer à ce sujet qu'une bromation d'un produit tel que le gaïacol par le brome donne 80 % de dérivé bromé en para et 20 % de dérivé bromé en ortho seulement.

L'objet principal de l'invention est donc un procédé permettant cette monobromation en ortho avec un rendement acceptable. Or, la Demanderesse s'est aperçu que le problème pouvait être résolu en utilisant un agent bromant spécifique.

Dans ce but, le procédé selon l'invention de préparation d'un dérivé mono-bromé en ortho du phénol est caractérisé en ce qu'on fait réagir un dérivé du phénol de formule

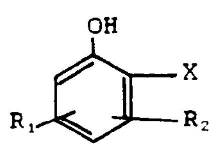
(1)

dans laquelle X représente OH, un radical alcoxy, $CH_2OH$, CHO, $CO_2R$, R étant un radical alkyle, alcényle, alkyloxy, ces radicaux pouvant être linéaires ou ramifiés, cycloalkyle, cycloalcényle, alkylcycloalcényle, benzyle, amine, terpénique, glycolique, $R_1$ et $R_2$ sont identiques ou différents et représentent l'hydrogène, un halogène, un radical alkyle, acyle ou alcoxy et avec comme condition que la position en ortho du OH un radical alcoxy soit libre avec un dérivé d'une amine présentant au moins une liaison N-Br.

Dans ces conditions, on obtient un dérivé monobromé en ortho avec un rendement d'au moins 60 %.

D'autres caractéristiques, détails et avantages de l'invention seront mieux compris à la lecture de la description et d'un exemple concret qui vont suivre.

Le produit de départ est un dérivé du phénol répondant à la formule 1 donnée ci-dessus.

L'invention s'applique particulièrement bien aux produits pour lesquels $R_1$ et $R_2$ sont en position méta et aussi à ceux pour lesquels $R_1$ et $R_2$ représentent tous les deux l'hydrogène.

On notera, que comme produit de départ particulièrement intéressant on peut utiliser ceux pour lesquels X est un radical alcoxy, et entre autres ceux pour lesquels $R_1$ et $R_2$ sont l'hydrogène, notamment parmi ceux-ci le gaïacol ($X = OCH_3$).

Comme autres produits de départ interessants, on peut mentionner aussi les salicylates, c'est-à-dire les produits pour lesquels X dans la formule (1) est un radical $CO_2R$, R étant tel que défini plus haut.

On peut citer à titre d'exemple les produits pour lesquels $R_1$ et $R_2$ sont tous les deux l'hydrogène et notamment les salicylates de n-hexyle, de benzyle, de méthyle, d'amyle, d'isoamyle, d'éthyl-2 hexyle, de cis-hexen-3 yle-1, de glycol, de prenyle, d'éthyl-2 butyle, de méthyl-2 pentyle, de monométhylamine, de triméthyl 3,3,5 cyclohexyle, d'isopropyl-2 cyclohexyle, de béta-isopropoxyéthyle, de bornyle.

Comme on l'a vu plus haut, l'agent bromant avec lequel on fait réagir le composé de formule 1 est un dérivé bromé d'une amine. On choisit plus spécialement les amines aliphatiques et en particulier les alkylamines. Les dérivés bromés de formules RNHBr, $RNBr_2$ ou RR′NBr pour lesquelles R et/ou R′ représentent un radical méthyle, éthyle, isopropyle, isobutyle, cyclohexyle et plus particulièrement tertiobutyle peuvent être citées.

La réaction est conduite en milieu solvant. Comme solvant on pourra utiliser les hydrocarbures aliphatiques halogénés, les hydrocarbures aromatiques halogénés ou non, les éthers.

On peut citer à ce sujet le tétrachlorure de carbone, le dichlorométhane, le chloroéthane, le dichlorobenzène.

Pour les éthers, on peut mentionner plus particulièrement l'éther isopropylique, l'oxyde de butyle, l'oxyde d'hexyle.

La réaction se fait généralement à une température voisine de 0°C.

La concentration en dérivé du phénol (produit de départ) est habituellement comprise entre 0,1 et 10 moles/l.

On utilise en général une quantité d'agent bromant comprise entre 0,8 et 1,2 équivalent de brome réactif.

La réaction se fait en principe en coulant l'agent bromant dans le mélange solvant-dérivé du phénol.

Une fois la réaction terminée, on peut séparer le produit du milieu réactionnel par tout moyen connu après, le cas échéant, traitement classique pour le passage de sa forme sel à la forme recherchée. Un exemple de traitement consiste à ajouter une solution acide au milieu réactionnel et à augmenter la température afin d'hydrolyser le précipité formé au cours de la réaction.

Un exemple concret mais non limitatif va maintenant être donné.

EXEMPLE

Dans un ballon tricol de 100 ml muni d'un thermomètre, d'un réfrigérant relié à un barboteur contenant de la soude et du sulfite de sodium en solution aqueuse, d'une ampoule à brome, d'une agitation magnétique, on place :

. 7,25 g de gaïacol,
. 75 ml d'éther isopropylique.

Le ballon est refroidi à 0°C, et en maintenant cette température, on ajoute goutte à goutte, par l'ampoule à brome, une solution de 6,75 g de N.N. dibromotertiobutylamine dans l'éther isopropylique.

3 heures après le début de l'addition, l'excès de brome réactif est détruit par une solution aqueuse de sulfite de sodium à 10 %, et 100 ml d'une solution d'acide sulfurique 2N sont ajoutés. Ce tout est porté à 50°C afin d'hydrolyser le précipité formé au cours de la réaction.

Après neutralisation, la phase organique est décantée, lavée et séchée.

Les résultats sont les suivants :

taux de transformation du gaïacol : 80 %,
rendement par rapport au produit transformé en bromo-6 gaïacol : 75 %,

Bien entendu, l'invention n'est nullement limitée aux modes de réalisation décrits qui n'ont été donnés qu'à titre d'exemples. En particulier, elle comprend tous les moyens constituant des équivalents techniques des moyens décrits ainsi que leurs combinaisons si celles-ci sont mises en oeuvre dans le cadre de la protection comme revendiquée.

## Revendications

1. Procédé de préparation d'un dérivé monobromé en ortho du phénol caractérisé en ce qu'on fait réagir un dérivé du phénol de formule :

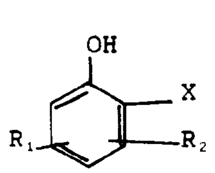

(1)

dans laquelle X représente OH, un radical alcoxy, $CH_2OH$, CHO, $CO_2R$ R étant un radical alkyle, alcényle, alkyloxy, ces radicaux pouvant être linéaires ou ramifiés, cycloalkyle, cycloalcényle, alkylcycloalcényle, benzyl, amine, terpénique, glycolique, $R_1$ et $R_2$ sont identiques ou différents et représentent l'hydrogène, un halogène, un radical alkyle, acyle ou alcoxy et avec comme condition que la position en ortho du OH soit libre ; avec un dérivé d'une amine présentant au moins une liaison N-Br.

2. Procédé selon la revendication 1 caractérisé en ce que le dérivé d'une amine précipité est un dérivé d'une amine aliphatique plus particulièrement d'une alkylamine.

3. Procédé selon la revendication 1 ou 2 caractérisé en ce qu'on fait réagir un dérivé du phénol de formule (1) dans laquelle $R_1$ et $R_2$ sont en position méta.

4. Procédé selon l'une des revendications 1 à 3, caractérisé en ce qu'on fait réagir un dérivé du phénol de formule (1) dans laquelle $R_1 = R_2 = H$.

5. Procédé selon l'une des revendications 1 à 4, caractérisé en ce qu'on fait réagir un dérivé du phénol de formule (1) dans laquelle X est un radical alcoxy.

6. Procédé selon l'une des revendications précédentes, caractérisé en ce que la réaction est conduite dans un solvant choisi dans le groupe comprenant les hydrocarbures aromatiques halogénés ou non, les éthers, les hydrocarbures aliphatiques halogénés.

## Patentansprüche

1. Verfahren zur Herstellung eines in ortho-Stellung monobromierten Phenolderivats, dadurch gekenn zeichnet, daß man ein Phenolderivat der Formel:

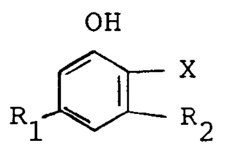

(1)

in der X OH, einen Alkoxyrest, $CH_2OH$, CHO, $CO_2R$ bedeutet, wobei R ein geradkettiger oder verzweigter Alkyl-, Alkenyl- oder Alkyloxyrest, oder ein Cycloalkyl-, Cycloalkenyl-, Alkylcycloalkenyl-, Benzyl-, Amin-, Terpen-, Glycolrest ist, $R_1$ und $R_2$ gleich oder verschieden sind und Wasserstoff, ein Halogen, einen Alkyl-, Acyl- oder Alkoxyrest bedeuten und unter der Bedingung, daß die ortho-Stellung zu OH frei ist, mit einer mindestens eine N-Br-Bindung aufweisenden Aminverbindung reagieren läßt.

2. Verfahren nach Anspruch 1, dadurch gekennzeichnet, daß die ausgefällte Aminverbindung ein Derivat eines aliphatischen Amins insbesondere eines Alkylamins ist.

3. Verfahren nach Anspruch 1 oder 2, dadurch gekennzeichnet, daß man eine Phenolverbindung der Formel (1) reagieren läßt, in der $R_1$ und $R_2$ in der meta-Stellung sind.

4. Verfahren nach einem der Ansprüche 1 bis 3, dadurch gekennzeichnet, daß man eine Phenolverbindung der Formel (1) reagieren läßt, in der $R_1 = R_2 = H$ ist.

5. Verfahren nach einem der Ansprüche 1 bis 4, dadurch gekennzeichnet, daß man die Phenolverbindung der Formel (1) reagieren läßt, in der X ein Alkoxyrest ist.

6. Verfahren nach einem der vorhergehenden Ansprüche, dadurch gekennzeichnet, daß die Reaktion in einem Lösungsmittel durchgeführt wird, das aus der Gruppe der gegebenenfalls halogenierten aromatischen Kohlenwasserstoffe, der Ether und der halogenierten aliphatischen Kohlenwasserstoffe ausgewählt ist.

## Claims

1. Process for preparing an ortho-monobromo derivative of phenol, characterised in that a phenol derivative of formula:

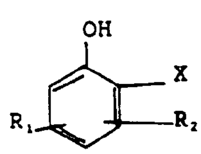

(1)

in which X denotes OH or an alkoxy, $CH_2OH$, CHO or $CO_2R$ radical, R being an alkyl, alkenyl or alkoxy radical, it being possible for these radicals to be linear or branched, or a cycloalkyl, cycloalkenyl, alkylcycloalkenyl, benzyl, amine, terpene or glycol radical, and $R_1$ and $R_2$ are identical or different and denote hydrogen, a halogen or an alkyl, acyl or alkoxy radical, and with the proviso that the position ortho to the OH is free, is reacted with a derivative of an amine possessing at least one N-Br bond.

2. Process according to claim 1, characterised in that the derivative of an amine precipitated is a derivative of an aliphatic amine, and more especially of an alkylamine.

3. Process according to claim 1 or 2, characterised in that a phenol derivative of formula (1) in which $R_1$ and $R_2$ are in the meta position is reacted.

4. Process according to one of claims 1 to 3, characterised in that a phenol derivative of formula (1) in which $R_1 = R_2 = H$ is reacted.

5. Process according to one of claims 1 to 4, characterised in that a phenol derivative of formula (1) in which X is an alkoxy radical is reacted.

6. Process according to one of the preceding claims, characterised in that the reaction is conducted in a solvent chosen from the group comprising halogenated or unhalogenated aromatic hydrocarbons, ethers and halogenated aliphatic hydrocarbons.